Europäisches Patentamt

European Patent Office

Office européen des brevets

(11) Publication number: **0 435 623 A1**

## EUROPEAN PATENT APPLICATION

(21) Application number: **90314177.8**

(22) Date of filing: **21.12.90**

(51) Int. Cl.⁵: **G01N 33/28**

(30) Priority: **27.12.89 GB 8929211**

(43) Date of publication of application:
**03.07.91 Bulletin 91/27**

(84) Designated Contracting States:
**AT CH DE GB LI**

(71) Applicant: **CASTROL LIMITED**
**Burmah House Pipers Way**
**Swindon Wiltshire SN3 1RE(GB)**

(72) Inventor: **Preston, William Hugh "Wansbeck"**
**Bethesda Street**
**Upper Basildon Berkshire(GB)**

(74) Representative: **Claisse, John Anthony, Dr.**
**97 Portway**
**Wells Somerset BA5 2BR(GB)**

(54) Monitoring apparatus.

(57) Apparatus for monitoring the condition of a fluid consists of an optical sensor (1) for providing an output indicative of the light obscuration caused by the fluid, a dielectric constant sensor (2), and an indicator (3) indicative of fluid condition which is responsive to outputs from the optical and dielectric constant sensors (1 and 2). The combination of the outputs from the two types of sensor is preferably effected using a microprocessor. The invention is particularly of use in monitoring the condition of used engine oil, for example from an internal combustion engine.

# MONITORING APPARATUS

This invention concerns apparatus for monitoring the condition of fluids, for example oil.

The repeated use of many fluids results in their deterioration. Lubricating oil in internal combustion engines, for example, is subjected to high temperatures and pressures during which the oil becomes contaminated with combustion products from the fuel and it undergoes chemical change as a result of oxidation by air at the high temperatures experienced by the oil in an engine. Thus the concentration of suspended particles resulting from combustion and other sources tends to increase, and the level of oxidation products of the oil increases, these leading to corrosion and/or engine wear.

These undesirable effects are reduced by regular oil changes which engine manufacturers usually recommend to be at regular intervals of time or of distance travelled. However, changing engine oil at such intervals does not take into account the very different operating cycles to which the oil in a particular engine has been subjected, and therefore the amount of suspended particles and/or the degree of oxidation which has, in fact, occurred. Thus oil in a particular engine may be unsatisfactory after only a very short period, and well within the recommended period for it to be changed, when subjected to certain engine cycles, whereas the same oil in the same engine may still have useful life remaining long after the recommended time or distance for changing it.

Visual observation indicates empirically that the oil might need to be changed, but it doesn't indicate oxidative changes in the oil. Furthermore, the consequences of the darkening of engine oil differ significantly between engines fuelled by gasoline and by diesel fuel.

Devices have been proposed hitherto for estimating the condition of engine oil. One group of such devices operates by detecting changes in the dielectric constant of a thin film of the oil resulting from oxidation of the oil. U.S. Patent Specification 3182255 describes such a device, another device working on the same principle being described in U.S. Patent Specification 3331019. Another group of devices operates by detecting changes in the degree of light obscuration caused by particles suspended in the oil.

Both of these hitherto proposed types of device have disadvantages. Dielectric constant does not indicate satisfactorily the level of contaminant particles, and light obscuration does not indicate the level of oxidative changes which have taken place in the oil. Furthermore, an acceptable level of suspended particles combined with an acceptable level of change in dielectric constant may result in the oil causing unacceptably high engine wear and/or corrosion which would not be expected from either measurement alone.

According to the present invention there is provided apparatus for monitoring the condition of a fluid, the apparatus comprising optical sensor means for providing an output indicative of the light obscuration caused by the fluid, dielectric constant sensor means for providing an output indicative of the dielectric constant of the fluid, and fluid condition indicator means responsive to the outputs from the optical sensor means and to the dielectric constant sensor means to provide an indication of the condition of the fluid.

Apparatus according to the present invention can be used to monitor the condition of a variety of fluids from various sources, but it will generally be used to monitor non-aqueous fluids, and especially oils. A particularly preferred use is for the monitoring of the condition of the oil from internal combustion engines.

In order to compensate for differences in the source or the type of fluid to be monitored, the outputs from either or both of the sensors is preferably adjustable to provide respective ranges of output which the fluid condition indicator means can interpret as ranging from acceptable to unacceptable fluid condition. Alternatively, the fluid condition indicator means can be adjustable to compensate for differences in the levels of the outputs from either or both of the sensor means according to the source or type of fluid to be monitored.

In the case of oils used in internal combustion engines, the light obscuration of acceptable oil from engines fuelled by gasoline will be different from that of acceptable oil from a diesel engine. This will necessitate changing the range of acceptability of light obscuration, for example by changing the responsiveness of the condition indicator means to the output from the optical sensor means or by changing the magnitude of the output from the optical sensor means before it is passed to the condition sensor means.

Different types of oil have inherently different values of dielectric constant. Thus new ester-based oils often have dielectric constants which would be considered unacceptable for a hydrocarbon-based oil. It is therefore preferred that apparatus of the present invention be capable of compensating for such differences by changing the magnitude of the output from the dielectric constant sensor means, or by changing the responsiveness of the condition indicator means, for example in a similar manner to that used for the output from the optical sensor means. In general, the output from the dielectric constant sensor means will be adjusted for changes in temperature of the fluid being tested and ambient humidity levels. This can be effected in known manner by changing the output from the sensor to compensate for changes in temperature, or by changing the temperature of the fluid being tested.

Apparatus of the present invention is capable of sensing two different properties of the fluid to be monitored. While both properties will usually be sensed, there may be cases where the output from one sensor means alone indicates that the fluid is satisfactory. Thus in the case of oil from internal combustion engines, if the degree of optical obscuration is sufficiently low, the oil may be passed as satisfactory without using the output from the dielectric constant sensor means. However, if the light obscuration is unsatisfactory, the condition indicator means then uses the output from the dielectric constant sensor means to establish whether the oil is satisfactory, a low output indicating acceptability and a high output unacceptability.

In a particularly preferred embodiment of the present invention, the outputs from the two sensor means are combined to provide an indication of fluid condition based on a combination of the two outputs. The significance of the respective outputs from the two sensor means can then be adjusted as desired to provide an indication of fluid condition weighted to take greater or lesser account of one or other of the outputs.

The condition indicator means can take any convenient form. It can be in the form of a visual display unit, although it is particularly preferred in the case of apparatus of the invention to be used to monitor engine oil from cars taken to garages to be able to provide a printed record, for example to give to the driver.

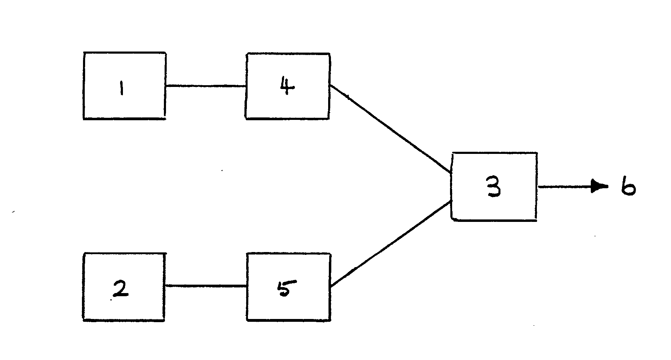

An embodiment of the present invention will now be described, by way of example, with reference to the accompanying diagrammatic drawing, the single figure of which showing a block diagram of the embodiment.

Referring to the drawing, an optical sensor 1 and a dielectric constant sensor 2 are each connected to a fluid condition indicator 3 via an attenuator 4 or 5 respectively. The optical sensor 1 and the dielectric constant sensor 2 are of hitherto proposed construction, and the indicator 3 contains electronic circuits which respond to the outputs from the two sensors 1 and 2 after passage through the attenuators 4 and 5, the circuits being such as to provide the response described in detail hereinafter. The indicator 3 provides an indication of oil condition as indicated by the arrow 6.

Attenuation of the outputs from the sensors 1 and 2 is preferably effected by a suitably programmed microprocessor, as is the combination of the attenuated outputs themselves.

In use, the attenuator 4 associated with the optical sensor 1 is adjusted according to whether the oil to be tested is from an engine fuelled by gasoline or by diesel fuel, and the attenuator 5 associated with the dielectric constant sensor 2 is adjusted according to the type of oil being tested, e.g. hydrocarbon or ester.

Samples of the oil to be tested are then supplied to the optical sensor 1 and to the dielectric sensor 2. The outputs from the sensors 1 and 2, attenuated if and as required, are then passed to the indicator 3.

If the degree of light obscuration of the oil sample in the optical sensor 1 as measured by the output fed to the indicator 3 is below a predetermined level, i.e. the light transmission is relatively high, the indicator 3 shows the oil to be satisfactory for continued use irrespective of the type of oil being tested or the fuel used, subject to the reservation given below. If, however, the output from the sensor 1 passed to the indicator 3 indicates that the degree of light obscuration is above a predetermined level, i.e. low light transmission, the indicator 3 then compares the output from the dielectric sensor 2 against a predetermined value to provide an indication as to whether the oil should continue to be used depending upon whether the oil is from a diesel or a gasoline fuelled engine.

Oil from a diesel fuelled engine is then passed for further use if its dielectric constant is below a certain value, and as unsatisfactory if above that value. A high dielectric value coupled with a high light transmission could indicate dilution with fuel, and dielectric constant will usually be measured for oil from diesel engines even if light obscuration is satisfactory.

A high dielectric constant for oil from a gasoline fuelled engine also indicates that it is unsatisfactory for further use. However, low values of dielectric constant for such oils is usually taken to indicate that the oil is borderline.

Intermediate values of dielectric constant are usually taken to indicate that the oil is probably unsatisfactory for further use.

It should be appreciated that the degree of light obscuration and the value of dielectric constant which constitute acceptability of an oil, or indeed of any other fluid, are arbitrary and they do not form part of the present invention.

In an alternative embodiment of the present invention, instead of the indicator 3 providing merely an indication of the tested oil passing or failing the test, it provides an indication of oil condition on a continuous scale. This has the advantage of providing an indication of how long it will be before an acceptable oil becomes unacceptable. The relative weights to be placed on the outputs from the sensors can, of course, be adjusted as desired. As with the previous embodiment, attenuation of the outputs of the sensors and their combination is preferably effected using a microprocessor. Weighting of the outputs from the sensors to provide an indication of oil condition on a continuous scale can also be effected using a microprocessor.

**Claims**

1. Apparatus for monitoring the condition of a fluid, the apparatus comprising optical sensor means for providing an output indicative of the light obscuration caused by the fluid, dielectric constant sensor means for providing an output indicative of the dielectric constant of the fluid, and fluid condition indicator means responsive to the outputs from the optical sensor means and to the dielectric constant sensor means to provide an indication of the condition of the fluid.

2. Apparatus according to claim 1, wherein the optical sensor means is adjustable to provide a range of outputs to the fluid condition indicator means dependant upon the fluid to be tested.

3. Apparatus according to claim 1 or claim 2, wherein the dielectric constant sensor means is adjustable to provide a range of outputs to the fluid condition indicator means dependant upon the fluid to be tested.

4. Apparatus according to any of the preceding claims, wherein the fluid condition indicator means only responds to the output from the dielectric constant sensor means if the output from the optical sensor means indicates the fluid condition to be unacceptable.

5. Apparatus according to any of the preceding claims, wherein the fluid condition indicator means provides an indication of acceptable and unacceptable fluid condition.

6. Apparatus according to any of the preceding claims wherein the fluid condition indicator means provides a printed indication of fluid condition.

7. Apparatus according to any of the preceding claims, wherein the fluid condition indicator means provides an indication of the condition of the fluid ranging from acceptable to unacceptable.

8. Apparatus according to any of the preceding claims for monitoring the condition of the oil from an internal combustion engine.

9. Apparatus according to claim 8, wherein the output from the optical sensor means is adjustable to compensate for differences in the fuel used in the engine from which the oil is to be monitored.

10. Apparatus according to claim 8 or claim 9, wherein the output from the dielectric constant sensor means is adjustable to compensate for differences in the type of oil used in the engine from which the oil is to be monitored.

11. A method of monitoring the condition of engine oil, the method comprising measuring the light obscuration of the oil, measuring the dielectric constant of the oil, and combining the measurements of light obscuration and dielectric constant to provide an indication of the condition of the oil.

12. A method according to claim 11, wherein the combination of the measurements is effected using a microprocessor.

13. A method according to claim 11 or claim 12, wherein the respective measurements are weighted to provide an indication of oil condition on a continuous scale.

# European Patent Office

**EUROPEAN SEARCH REPORT**

Application Number

**EP 90 31 4177**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PATENT ABSTRACTS OF JAPAN, vol. 10, no. 271 (P-497)[2327], 16th September 1986; & JP-A-61 95 245 (TOKYO TATSUNO CO.) 14-05-1986 * Whole document * | 1-3,8-11 | G 01 N 33/28 |
| | — — — | | |
| A | US-A-3 526 127 (MOBIL OIL) * Abstract; column 3, lines 56-61,72-74; figure 1 * | 1,5-8,12 | |
| | — — — | | |
| A | CH-A-3 696 12 (ISTVAN NAGY) * Page 2, lines 110-114; claims 1,2; figure 2 * | 1,2,5,8,13 | |
| | — — — | | |
| A | US-A-4 699 509 (KAMIYA) * Abstract; column 9, lines 12-15; figure 19 * | 1,5,7 | |
| | — — — — — | | |

TECHNICAL FIELDS SEARCHED (Int. Cl.5)

G 01 N

The present search report has been drawn up for all claims

| Place of search | Date of completion of search | Examiner |
|---|---|---|
| The Hague | 08 April 91 | KEMPF G.V. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document